# EUROPEAN PATENT APPLICATION

(11) **EP 2 153 775 A1**
(43) Date of publication of application: **17.02.2010**
(21) Application number: 08752232.2
(22) Date of filing: 29.04.2008
(51) Int. Cl.: A61B 5/151, A61B 5/1473, A61B 5/1486, A61B 5/15, A61B 5/157, G01N 27/327, G01N 27/416, G01N 33/48

(54) **ANALYSIS INSTRUMENT**

(30) Priority: 29.04.2007 JP 2007120396
(71) Applicant: Arkray, Inc., Kyoto-shi, Kyoto 601-8045 (JP)
(72) Inventor: DOI, Shigeru, Kyoto-shi Kyoto 601-8045 (JP)
(74) Representative: HOFFMANN EITLE
(86) International application number: PCT/JP2008/058222
(87) International publication number: WO 2008/136473

(57) **Abstract**

This invention is regard to an analysis tool comprising a hole 23 through which laser beam to be emitted to skin travels for issuing bodily fluid, and a cover 25 which closes an opening from 23B which the laser beam in the hole 23 enters and through which the laser beam can pass. For example the analysis tool 2 further comprising a plurality of electrodes 20, 21 which are laminated one another in a state where they are electrically insulated from each other, and which have through holes 20A, 21A for defining the hole 23. Desirably the analysis tool further comprising a discharge passage 26 through which gas in the hole 23 is discharged outside.

## Description

### TECHNICAL FIELD

The present invention relates to an analysis tool for analyzing specific ingredient (such as glucose, cholesterol and lactic acid) in the bodily fluid issued from a skin by irradiation of laser beam.

### BACKGROUND TECHNOLOGY

When measuring concentration of glucose or the like in blood, a method of utilizing a single-use analysis tool is employed as a simple method (see patent document 1, for example). As the analysis tool, there is one capable of carrying out analysis electrochemically or optically.

A sample such as blood can be obtained by incising a skin using a lancet, for example. It is general to impale a skin with a puncture needle as the lancet, but there is also a lancet capable of issuing blood from a skin by irradiating the skin with laser beam (see patent document 2, for example).

For example, the laser lancet includes a laser diode for emitting laser beam, and a condensing lens for collecting laser beams.

However, when a skin is irradiated with laser beam to issue blood, foreign matter such as fume or dust from a skin adheres to the condensing lens or a light emitting portion of the laser diode in some cases. When such a case occurs, a skin cannot appropriately be irradiated with laser beam, and sufficient amount of blood cannot be issued from a skin.

In order to protect the emitting surface of laser beam of the condensing lens, it is proposed to provide a protection cover (see patent document 3, for example). According to the structure of providing the protection cover, although it is possible to protect the condensing lens or the laser diode, it is necessary to clean the protection cover, maintenance is required, and a user is required to do a troublesome operation. Further, if a user neglects to do the maintenance, a skin cannot be appropriately irradiated with laser beam. It is possible to use a single-use protection cover, but in this case, the replacing operation of the protection cover is required, and a load on a user is increased.

Patent Document 1: Japanese Patent Publication No. H8-10208
Patent Document 2: Japanese Patent Application Laid-open No .H4-314428 Patent Document 2: International Application Laid-open No. WO98/47435

### DISCLOSURE OF THE INVENTION

### PROBLEMS TO BE SOLVED BY THE INVENTION

It is an object of the present invention to appropriately irradiate a skin with laser beam without putting a load on a user when issuing bodily fluid such as blood from the skin using a lancet.

### MEANS FOR SOLVING THE PROBLEM

The present invention provides an analysis tool comprising a hole through which laser beam to be emitted to skin travels for issuing bodily fluid, and a cover which closes an opening from which the laser beam in the hole enters and through which the laser beam can pass.

The analysis tool of the invention further includes a plurality of electrodes which are laminated one another in a state where they are electrically insulated from each other, and which have through holes for defining the hole. The analysis tool of the invention may further include a discharge passage through which gas in the hole is discharged outside. For example, the discharge passage is provided by forming, in an insulation layer interposed between the electrode and the cover, a slit which is in communication with the hole.

For example, the hole is for applying capillary action to suck bodily fluid from a skin.

The analysis tool of the invention further includes a reagent section formed on an inner surface of the hole.

The analysis tool of the invention may further include a passage through which bodily fluid sucked in the hole moves, and a reagent section formed in the passage.

The analysis tool of the invention may further include a substrate which supports the cover and which is provided with a plurality of electrodes. In this case, it is preferable that the analysis tool further includes a spacer which is interposed between the substrate and the cover, and which defines the passage.

### BRIEF DESCRIPTION OF THE DRAWINGS

Fig. 1 is an overall perspective view showing an example of an analysis apparatus using an analysis tool according to the present invention.
Fig. 2 is a sectional view taken along the line II-II in Fig. 1.
Fig. 3 is an overall perspective view showing an example of a biosensor according to the invention.
Fig. 4 is a partially exploded perspective view of the biosensor shown in Fig. 3.
Fig. 5 is a sectional view taken along the line V-V in Fig. 3.
Fig. 6 is a sectional view taken along the line VI-VI in Fig. 3.
Fig. 7 is a sectional view showing an essential portion for explaining a connector and a lasing mechanism in the analysis apparatus shown in Fig. 1.
Figs. 8A to 8C are sectional views showing an essential portion for explaining a sensor supply mechanism in the analysis apparatus shown Fig. 1.
Figs. 9A and 9B are sectional views showing an essential portion for explaining a sensor detection mechanism in the analysis apparatus shown Fig. 1.
Fig. 10 is a sectional view for explaining another example of the biosensor.
Fig. 11 is an overall perspective view for explaining another example of the biosensor.
Fig. 12 is a sectional view taken along the line XII-XII in Fig. 11.
Fig. 13 is an exploded perspective view of the biosensor shown in Fig. 11.
Fig. 14 is an overall perspective view for explaining another example of the biosensor.
Fig. 15 is a sectional view taken along the line XV-XV in Fig. 14.
Fig. 16 is an exploded perspective view of the biosensor shown in Fig. 14.
Fig. 17 is an overall perspective view for explaining another example of the biosensor.
Fig. 18 is a sectional view taken along the line XVIII-XVIIT in Fig. 17.
Fig. 19 is an exploded perspective view of the biosensor shown in Fig. 17.

### DESCRIPTION OF REFERENCE SYMBOLS

- 2, 8A, 8B, 8C: biosensor (analysis tool)
- 20, 81A, 82B, 82C: working electrode (electrode)
- 21, 82A, 82B, 82C: counter electrode (electrode)
- 23: capillary (hole of analysis tool)
- 23B: opening (of capillary)
- 24, 86A, 86B, 8C: reagent section
- 25, 25', 84A, 84B, 85C: cover
- 26: discharge passage
- 28: insulation layer
- 28A: slit (of insulation layer)
- 85A, 85B, 86C: passage

### PREFERRED EMBODIMENT OF THE INVENTION

An analysis tool according to the present invention will be described together with an analysis apparatus using the analysis tool with reference to the drawings.

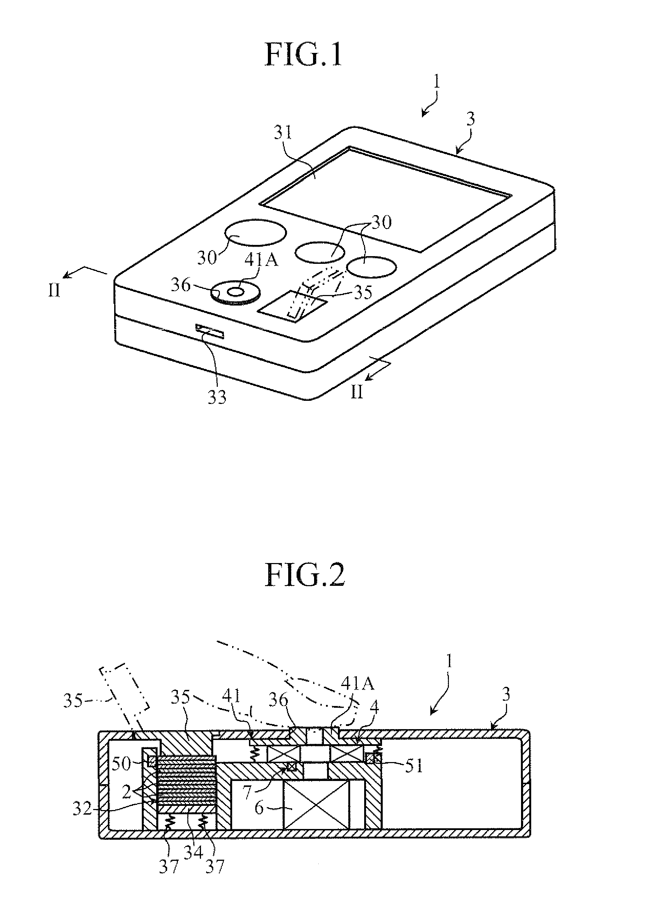

An analysis apparatus 1 shown in Figs. 1 and 2 is for analyzing a sample by an electrochemical method using biosensors 2. The analysis apparatus 1 is constituted as a portable type apparatus that can easily be carried. The analysis apparatus 1 accommodates therein a plurality of biosensors 2, and includes a casing 3, a connector 4, a sensor supply mechanisms 50 and 51, a lasing mechanism 6 and a sensor detection mechanism 7.

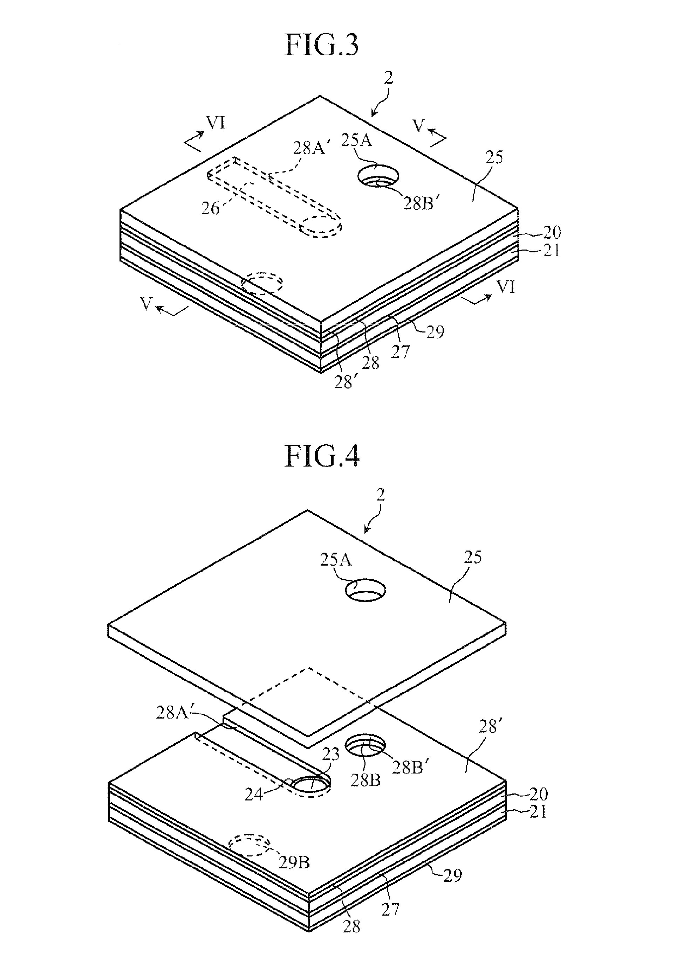

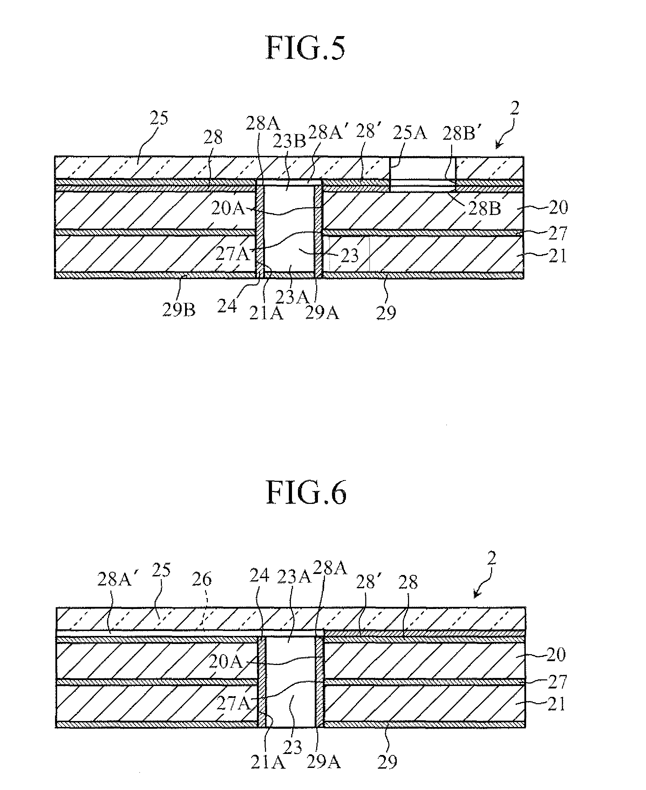

As shown in Figs. 3 to 6, the biosensors 2 are constituted as single-use biosensors. The biosensor 2 are used for analyzing specific ingredient (such as glucose, cholesterol and lactic acid) in bodily fluid such as blood and interstitial fluid. The biosensor 2 is formed into a rectangular plate-like shape as a whole, and has a size of (2 to 10 mm) × (2 to 10 mm) × (0.5 to 2 mm) for example. The biosensor 2 includes a working electrode 20 and a counter electrode 21 which are laminated on each other, and further includes a capillary 23, a reagent layer 24, a cover 25 and a discharge passage 26.

The working electrode 20 and the counter electrode 21 apply voltage to bodily fluid introduced into the capillary 23, and are utilized to measure response current at that time. The working electrode 20 and the counter electrode 21 include through holes 20A and 21A and are formed into the same or almost the same shape. The through holes 20A and 21A define the capillary 23, and are formed into a circle having a diameter of 0.2 to 1 mm at central portions of the working electrode 20 and the counter electrode 21. The working electrode 20 and the counter electrode 21 are made of conductive magnetic material such as nickel, and formed into a size of (2 to 10 mm) × (2 to 10 mm) × (0.2 to 1 mm).

An insulation layer 27 is interposed between the working electrode 20 and the counter electrode 21, and the working electrode 20 and the counter electrode 21 are bonded to each other through the insulation layer 27. A through hole 27A defining the capillary 23 is formed in a central portion of the insulation layer 27, and a thickness of the insulation layer 27 is formed into 20 to 100 µm by a known hot-melt sheet. A diameter of the through hole 27A is the same or almost the same as those of the through holes 20A and 21A of the working electrode 20 and the counter electrode 21.

Insulation layers, 28, 28' and 29 are formed in surfaces of the working electrode 20 and the counter electrode 21. The insulation layers 28 and 29 prevent bodily fluid from adhering the surfaces 20B and 21B of the working electrode 20 and the counter electrode 21. Like the insulation layer 27, these insulation layers 28 and 29 are formed by the known hot-melt sheet also. The insulation layers 28 and 29 are formed with through holes 28A and 29A which define the capillary 23. Diameters of the through holes 28A and 29A are the same or almost the same as the through holes 20A and 21A of the working electrode 20 and the counter electrode 21. The insulation layer 28' is formed with a slit 28A' which defines the discharge passage 26. The insulation layers 28, 28' and 29 are formed with holes 28B, 28B' and 29B through which the surface 20B or 21B of the working electrode 20 or the counter electrode 21 are formed. The measuring terminals 42 and 43 of the connector 4 can come into contact with the working electrode 20 or the counter electrode 21 through the holes 28B, 28B' and 29B.

The capillary 23 is for moving bodily fluid introduced from an opening 23A toward an opening 23B utilizing capillary action and for holding the bodily fluid therein. The capillary 23 permits laser beam from entering from the later-described lasing mechanism 6. The capillary 23 is defined by the through holes 20A, 21A and 27A to 29A of the working electrode 20, the counter electrode 21 and the insulation layers 27 to 29.

The reagent layer 24 includes a reagent required for analysis of specific ingredient in bodily fluid, and covers an inner surface of the capillary 23. The reagent layer 24 includes electron transport material and oxyreductase, and is formed into a solid object which easily melts in bodily fluid. When bodily fluid is introduced into the capillary 23, the reagent layer 24 melts, and a liquid-phase reaction system including electron transport material, oxyreductase and bodily fluid is constituted in the capillary 23.

Material as the oxyreductase is selected depending upon kinds of specific ingredient to be analyzed. For example, when glucose is to be analyzed, glucose dehydrogenase (GDH) or glucose oxidase (GOD) can be used. Material as the electron transport material, ruthenium complex or iron complex can be used. Topically, [Ru(NH₃)₆]Cl₃ or K₃[Fe(CN)₆] can be used.

The cover 25 seals the opening 23B of the capillary 23. The cover 25 includes a through hole 25A. The through hole 25A and the holes 28B and 28B' of the insulation layers 28 and 28' are for exposing the working electrode 20 therefrom. The cover 25 covers the entire working electrode 20 by means of material through which laser beam can pass, e.g., transparent glass and PET.

The discharge passage 26 is defined by a slit 28A' of an insulation layer 28'. The slit 28A' is formed up to an edge of the insulation layer 28', and is connected to the capillary 23. That is, the discharge passage 26 can discharge gas in the capillary 23.

The casing 3 shown in Figs. 1 and 2 defines an outward appearance of the analysis apparatus 1, and includes a plurality of operation buttons 30, a display panel 31, a sensor accommodating portion 32 and a waste vent 33. The plurality of operation buttons 30 produce signals for carrying out the analysis operation, and for carrying out various setting operations (such as setting of analysis condition and input of ID of a subject). An analysis result, an error, operating procedure and an operating status at the time of setting operation are displayed on the display panel 31. The plurality of biosensors 2 are laminated and accommodated in the sensor accommodating portion 32. The sensor accommodating portion 32 includes a mounting portion 34 and a lid 35 which can open and close. The mounting portion 34 is biased upward by a coil spring 37 (toward the lid 35). A biosensor 2 that was used for analysis is discarded from the analysis apparatus 1 through the waste vent 33.

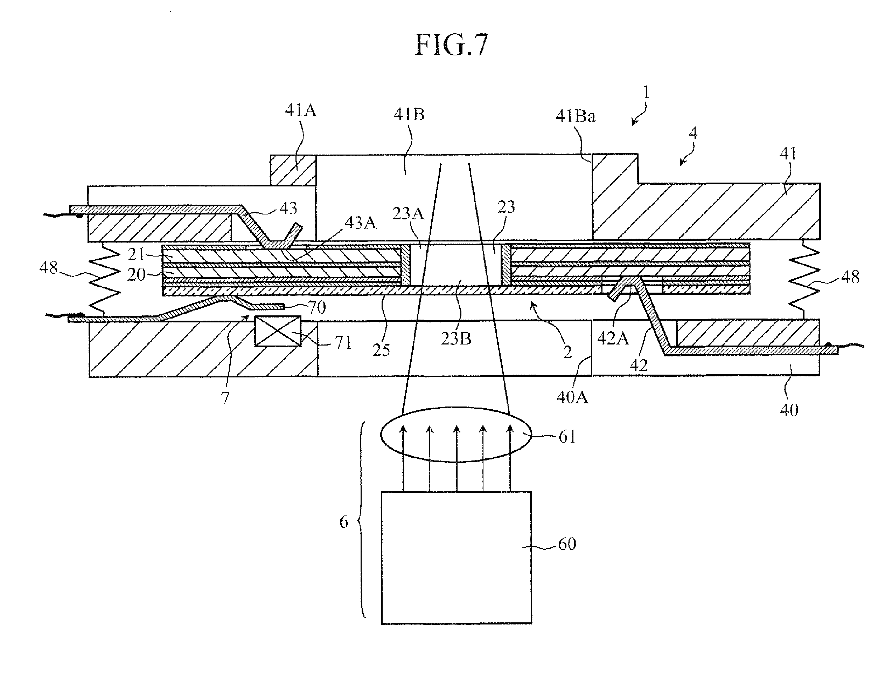

As shown in Fig. 7, the connector 4 holds a biosensor 2 to be analyzed, and applies voltage between the working electrode 20 and the counter electrode 21 of the biosensor 2. The connector 4 includes a fixed body 40, a movable body 41 and measuring terminals 42 and 43.

The fixed body 40 supports the measuring terminal 42, and includes a through hole 40A. The through hole 40A permits laser beam to travel from the lasing mechanism 6. The later-described sensor detection mechanism 7 (elastic body 70 and switch 71) are disposed in the fixed body 40.

The movable body 41 supports the measuring terminal 43. The movable body 41 is connected to the fixed body 40 through a coil spring 48, the movable body 41. is biased upward and the movable body 41 can vertically move. The movable body 41 includes a convex portion 41A and a through hole 41B. A skin such as a fingertip is pushed against the convex portion 41A when extracting bodily fluid, and the convex portion 41A is exposed from a through hole 36 (see Fig. 1) of the casing 3. That is, if a skin such as a fingertip is pushed against the convex portion 41A, the movable body 41 is moved downward. The through hole 41B permits laser beam to enter from the lasing mechanism 6, and the through hole 41B continuously extends to the convex portion 41A, and is in communication with outside of the apparatus at an end surface of the convex portion 41A. That is, the opening 41Ba functions as a bodily fluid extracting opening of the through hole 41B.

The measuring terminals 42 and 43 are constituted as leaf springs. The measuring terminals 42 and 43 apply voltage between the working electrode 20 and the counter electrode 21 of the biosensor 2. The measuring terminal 42 comes into contact with the working electrode 20, and the contact 42A projects upward. The measuring terminal 43 comes into contact with the counter electrode 21, and the contact 43A projects downward.

In the connector 4, the contact 42A of the measuring terminal 42 constituted as a leaf spring projects upward from the fixed body 40, and the contact 43A of the measuring terminal 43 projects downward from the movable body 41. Therefore, in the connector 4, the biosensor 2 can be held between the fixed body 40 and the movable body 41.

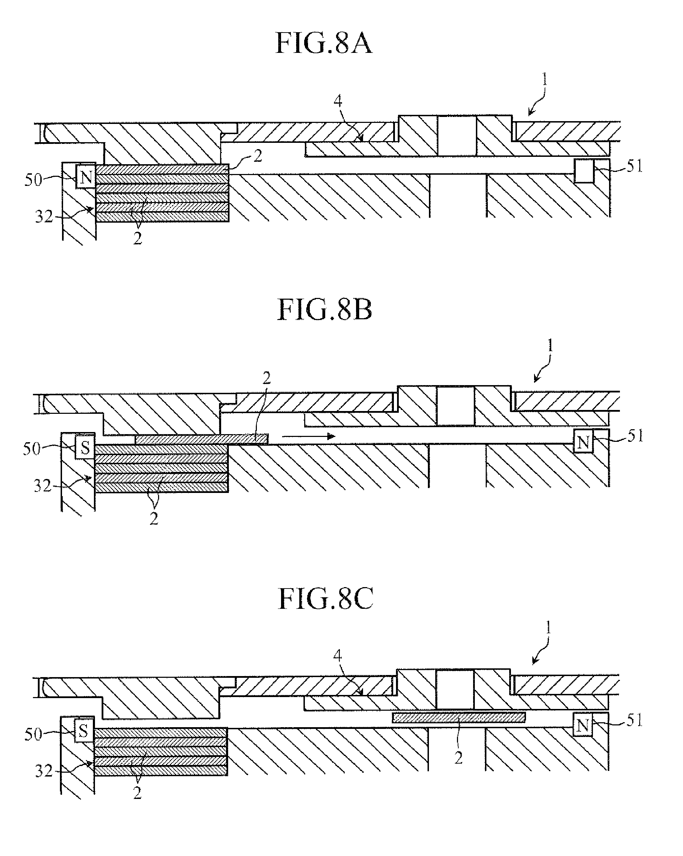

As shown in Figs. 8A to 8C, the sensor supply mechanisms 50 and 51 supply, to the connector 4, the uppermost one of the plurality of biosensors 2 laminated on the sensor accommodating portion 32. The sensor supply mechanisms 50 and 51 include electromagnets 50 and 51, respectively. The electromagnet 50 is provided adjacent to the sensor accommodating portion 32, and the electromagnet 51 is provided adjacent to the connector 4. The electromagnet 50 magnetizes the biosensor 2, and applies repulsion between the magnetized biosensor 2 and the electromagnet 50. The electromagnet 51 applies an attraction force between the magnetized biosensor 2 and the electromagnet 51.

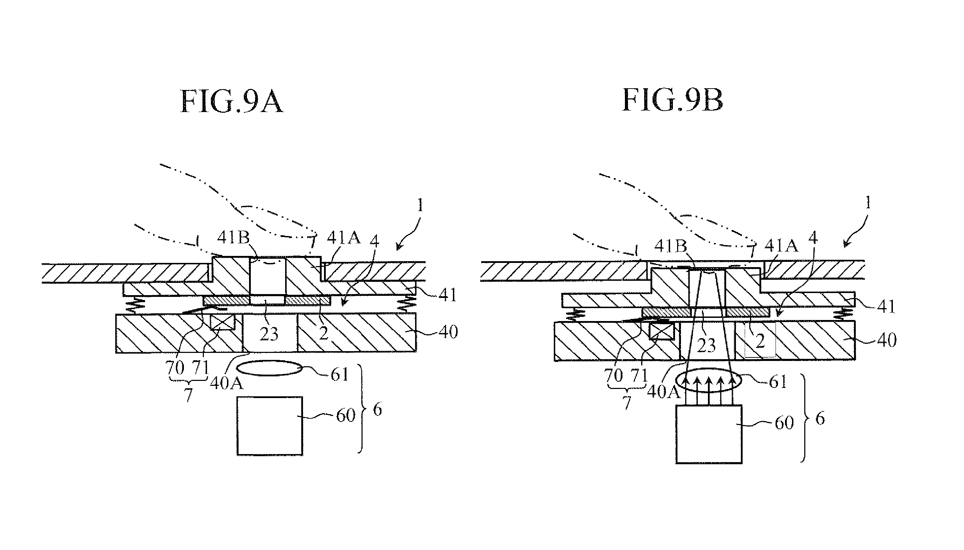

As shown in Figs. 9A and 9B, when issuing bodily fluid such as blood from a skin, the lasing mechanism 6 emits laser beam to be emitted to the skin. The lasing mechanism 6 includes a laser beam oscillator 60 such as a laser diode and a condensing lens 61.

As shown in Figs. 7, 9A and 9B, the sensor detection mechanism 7 is for detecting whether the biosensor 2 exists in a target position of the connector 4, and includes the elastic body 70 and the switch 71. The elastic body 70 is fixed to the fixed body 40 in the connector 4, and is short-circuited with the switch 71. The elastic body 70 turns the switch 71 ON when the movable body 41 moves downward. The switch 71 is for turning predetermined motion of the analysis apparatus 1 ON and OFF. When the switch 71 is ON, the switch 71 controls the laser beam oscillator 60 and emits laser beam.

The elastic body 70 may be fixed to the movable body 41. The elastic body 70 may have elasticity due to a shape other than a leaf spring or properties of material.

Next, operation of the analysis apparatus 1 will be described.

As shown in Figs. 8A to 8C, in the analysis apparatus 1, when a plurality of biosensors 2 are set in the sensor accommodating portion 32 or analysis is completed, the biosensors 2 are supplied to the connector 4 from the sensor accommodating portion 32 by the sensor supply mechanisms 50 and 51.

More concretely, in the sensor supply mechanisms 50 and 51, as shown in Fig. 8A, the biosensor 2 is magnetized by the electromagnet 50. In the illustrated example, in the electromagnet 50, the north pole is adjacent to the biosensor 2, a side of the biosensor 2 close to the electromagnet 50 is magnetized with south pole and a side of the biosensor 2 farther to the electromagnet 50 is magnetized with the north pole. At that time, no magnetic pole is generated in the electromagnet 51.

Next, as shown in Figs. 8B and 8C, the polarity of the electromagnet 50 is reversed and repulsion is generated between the biosensor 2 and the electromagnet 50. The polarity is generated in the electromagnet 51, and an attraction force is generated between the biosensor 2 and the electromagnet 50 as reversed polarity. The biosensor 2 is moved toward the connector 4 by the repulsion of the electromagnet 50 and the attraction force by the electromagnet 51, and the biosensor 2 is held by the connector 4. At that time, the measuring terminal 42 of the connector 4 comes into contact with the working electrode 20, and the measuring terminal 43 comes into contact with the counter electrode 21.

The sensor supply mechanisms 50 and 51 are not limited to those having the electromagnets 50 and 51, and may utilize a known actuator for example. In this case, in the biosensor 2, it is not always necessary that the working electrode 20 and the counter electrode 21 are made of magnetic material.

As shown in Figs. 9A and 9B, when analysis of specific ingredient in bodily fluid is to be carried out using the analysis apparatus 1, a skin such as a fingertip is pushed against the convex portion 41A of the movable body 41, and the movable body 41 is moved downward. With this, the elastic body 70 in the sensor detection mechanism 7 is moved downward together with the movable body 41 (biosensor 2). If the elastic body 70 moves downward, the elastic body 70 turns the switch 71 ON, and the power supply of the analysis apparatus 1 is turned ON. At that time, laser beam is emitted from the lasing mechanism 6.

As shown in Fig. 7, the biosensor 2 includes the capillary 23. The fixed body 40 and the movable body 41 include through holes 40A and 41B. Thus, a skin placed on the convex portion 41A is irradiated with laser beam emitted from the laser beam oscillator 60. When a skin is irradiated with laser beam, bodily fluid such as blood is issued from the skin. At that time, since the skin is pushed against the convex portion 41A, the skin is congested, and issuing phenomenon of bodily fluid such as blood is accelerated.

The biosensor 2 is mounted on the connector 4 of the analysis apparatus 1 such that the opening 23A of the capillary 23 sealed by the cover 25 is located on the incident side of the laser beam. That is, the biosensor 2 can suppress the contamination on a light-emitting surface of the condensing lens 61 or the laser beam oscillator 60 in the lasing mechanism 6 that may be caused by fume generated when a skin is irradiated with laser beam or by scattering of blood or skin. Therefore, a skin can appropriately be irradiated with laser beam.

The single-use biosensor 2 prevents contamination caused by fumes, blood or skin from adhering and thus, it is unnecessary to clean the condensing lens 61. Thus, a load on a user is reduced, and nonuniform values of laser output generated when the condensing lens 61 is cleaned can be suppressed.

Bodily fluid from a skin is introduced into the capillary 23 by capillary action generated in the capillary 23 of the biosensor 2. The reagent layer 24 is melted in the capillary 23, and the liquid-phase reaction system is constituted.

When the switch 71 is turned ON, voltage is applied between the measuring terminals 42 and 43 in the connector 4. With this, voltage is applied between the working electrode 20 and the counter electrode 21 and voltage is applied also to the liquid-phase reaction system. With this, specific ingredient such as glucose in the bodily fluid is reduced (electrons are taken out) by oxyreductase, and the electrons are supplied to the working electrode 20 through the electron transport material. An amount of electrons supplied to the working electrode 20 is measured as response current through the measuring terminals 42 and 43. In the analysis apparatus 1, concentration of specific ingredient such as glucose is calculated based on the response current. A result of the calculation is displayed on the display panel 31 shown in Fig. 1.

When the analysis of bodily fluid is completed, used biosensor 2 is discarded through the waste vent 33. Such biosensor 2 may be discarded automatically by a discarding mechanism provided in the analysis apparatus 1 or a user may discard the biosensor 2 manually by operating a lever.
When a used biosensor 2 is discarded, a new biosensor 2 is supplied to the connector 4 by the sensor supply mechanisms 50 and 51.

It is not always necessary to use a biosensor 2 that is previously accommodated in the analysis apparatus 1, and the biosensor 2 can be mounted on the connector 4 in the analysis apparatus 1 at the time of analysis.

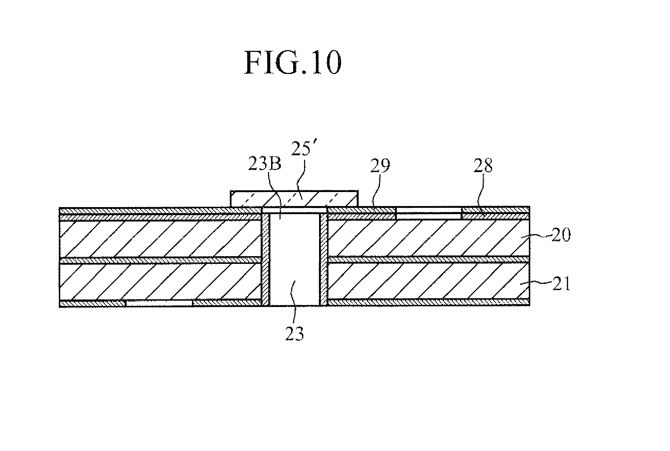

As shown in Fig. 10, the cover 25' need not cover the entire working electrode 20, and may selectively seal the opening 23B in the capillary 23.

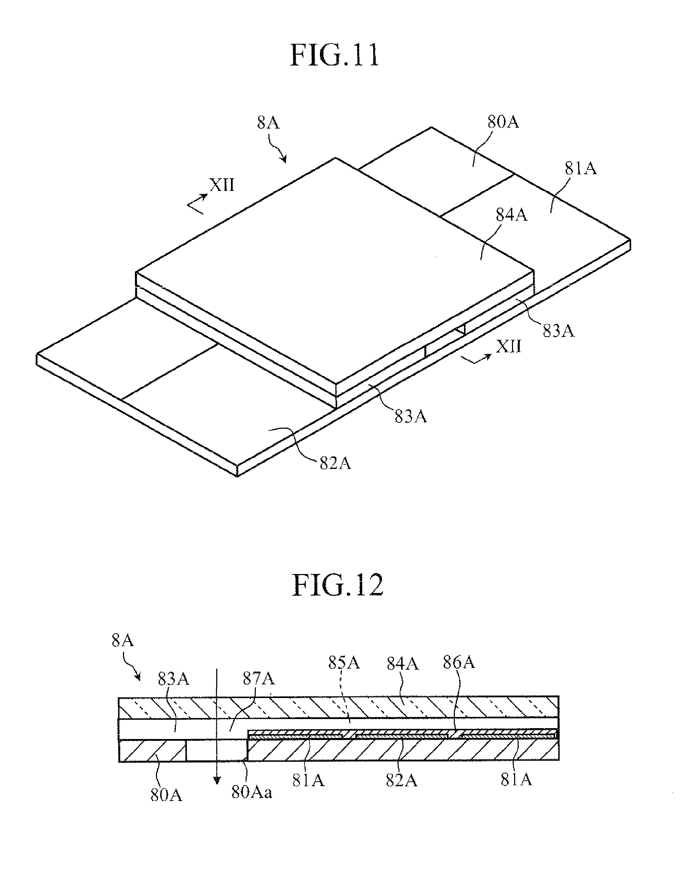

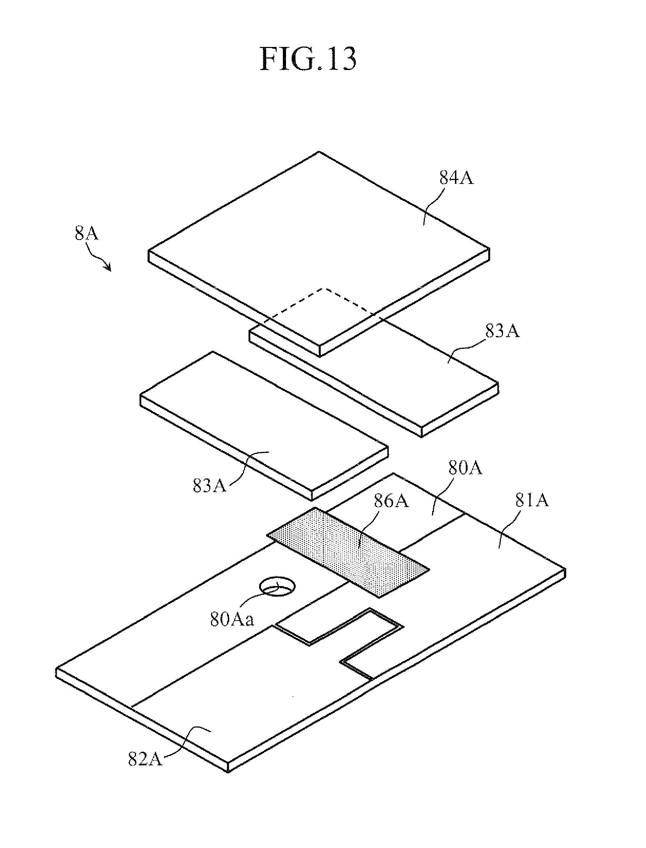

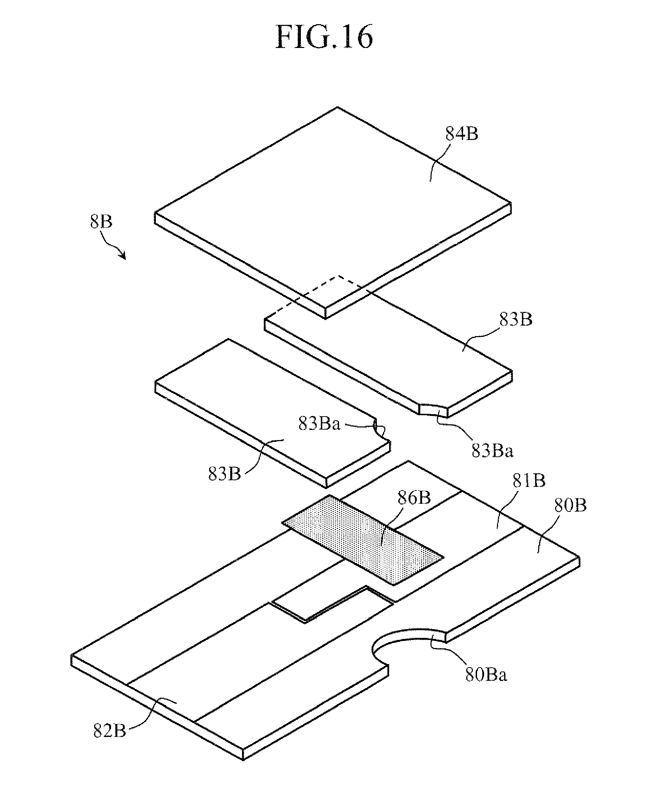

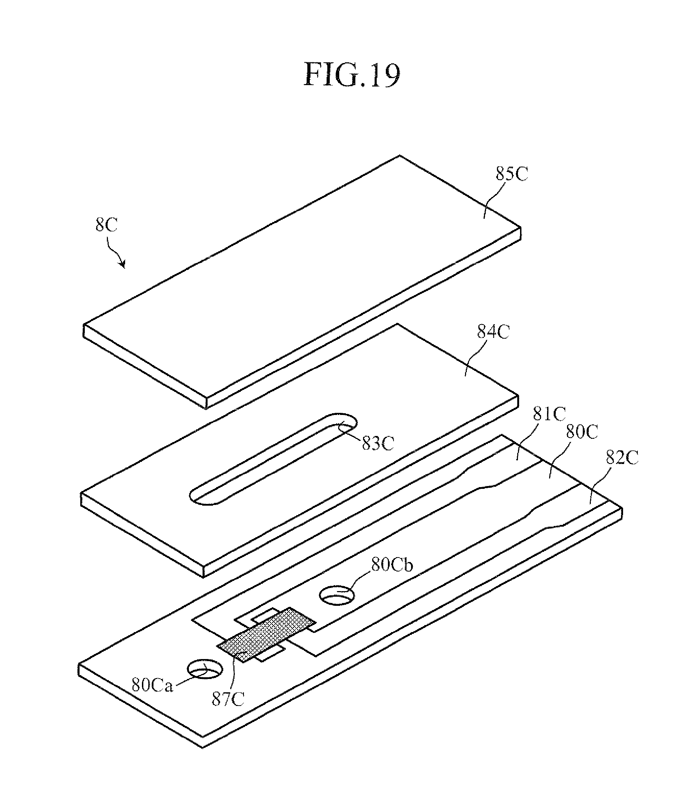

The present invention is not limited to the above-described embodiment, and the invention can variously be modified. For example, the invention can also be applied to a biosensor in which the working electrode and the counter electrode are provided on an insulative substrate as shown in Figs. 11 to 19.

According to a biosensor 8A shown in Figs. 11 to 13, an insulative substrate 80A is provided with a working electrode 81A and a counter electrode 82A, and a cover 84A is bonded to the insulative substrate 80A through a pair of spacers 83A which are disposed at a constant distance from each other. According to the biosensor 8A, a capillary 85A is provided between the pair of spacers 83A, and a reagent section 86A is formed in the capillary 85A.

The insulative substrate 80A is provided with a through hole 80Aa. The through hole 80Aa together with gaps of the pair of spacers 83A define a through hole 87A which permits laser beam to travel. The through hole 87A is closed with the cover 84A. The cover 84A is made of glass or PET and is transparent so that laser beam can pass through the cover 84A.

In the biosensor 8A also, the through hole 87A through which laser beam travels is closed with the cover 84A. This prevents the condensing lens 61 (see Fig. 7) in the lasing mechanism 6 from being contaminated.

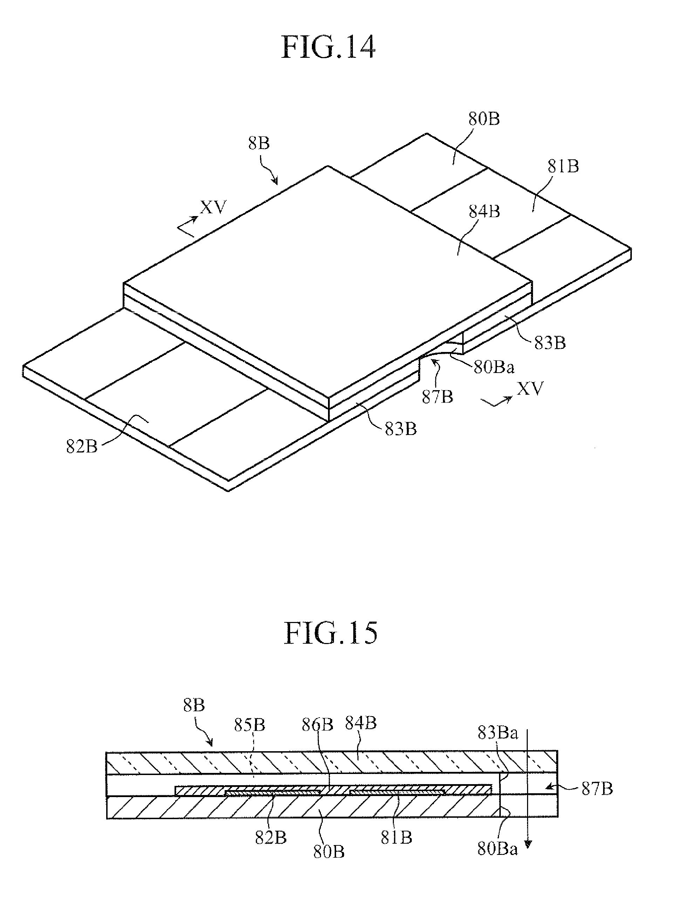

According to a biosensor 8B shown in Figs. 14 to 16, an insulative substrate 80B is provided with a working electrode 81B and a counter electrode 82B. A cover 84B is bonded to the insulative substrate 80B through a pair of spacers 83B which are separated from each other at a constant distance. In the biosensor 8B, a capillary 85B is provided between the pair of spacers 83B, and a reagent section 8B is formed in the capillary 85B.

The insulative substrate 80B is provided with a notch 80Ba. The notch 80Ba together with notches 83Ba of a pair of spacers 83B define a notch 87B which permits laser beam to travel. The notch 87B is closed with the cover 84B. The cover 84B is made of glass or PET and is transparent so that laser beam can pass through the cover 84B.

In the biosensor 8B also, the notch 87B through which laser beam travels is closed with the cover 84B. This prevents the condensing lens 61 (see Fig. 7) in the lasing mechanism 6 from being contaminated.

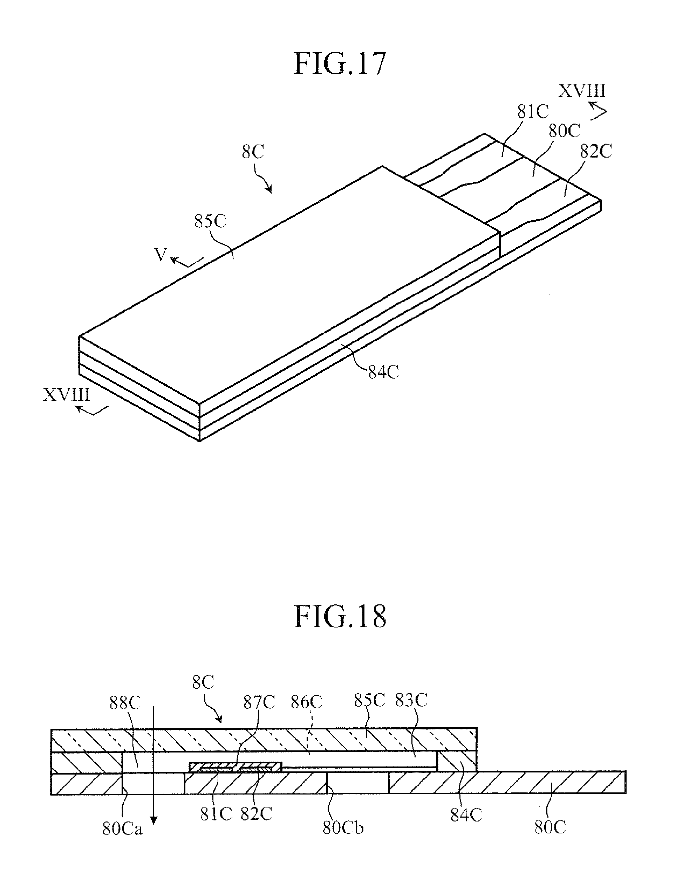

According to a biosensor 8C shown in Figs. 17 to 19, an insulative substrate 80C is provided with a working electrode 81C and a counter electrode 82C. A cover 85C is bonded to the insulative substrate 80C through a spacer 84C provided with a slit 83C. In the biosensor 8C, the capillary 86C is provided by the slit 83C, and a reagent section 87C is formed in the capillary 86C.

The insulative substrate 80C is provided with a through hole 80Ca. The through hole 80Ca together with the slit 83C define a through hole 88C which permits laser beam to travel. The through hole 86C is closed with the cover 85C. The cover 85C is made of glass or PET and is transparent so that laser beam can pass through the cover 8C.

The insulative substrate 80C is also provided with a through hole 80Cb. Gas in the capillary 86C is discharged from the through hole 80Cb.

In the biosensor 8C also, the through hole 88C through which laser beam travels is closed with the cover 85C. This prevents the condensing lens 61 (see Fig. 7) in the lasing mechanism 6 from being contaminated.

The present invention is not limited to a biosensor having the working electrode and the counter electrode, and can also be applied to an analysis tool such as a biosensor which carries out analysis of bodily fluid by colorimetry.

## Claims

1. An analysis tool comprising a hole through which laser beam to be emitted to skin travels for issuing bodily fluid, and a cover which closes an opening from which the laser beam in the hole enters and through which the laser beam can pass.

2. The analysis tool according to claim 1, further comprising a plurality of electrodes which are laminated one another in a state where they are electrically insulated from each other, and which have through holes for defining the hole.

3. The analysis tool according to claim 2, further comprising a discharge passage through which gas in the hole is discharged outside.

4. The analysis tool according to claim 3, wherein the discharge passage is provided by forming, in an insulation layer interposed between the electrode and the cover, a slit which is in communication with the hole.

5. The analysis tool according to claim 1, wherein the hole is for applying capillary action to suck bodily fluid from a skin.

6. The analysis tool according to claim 5, further comprising a reagent section formed on an inner surface of the hole.

7. The analysis tool according to claim 5, further comprising a passage through which bodily fluid sucked in the hole moves, and a reagent section formed in the passage.

8. The analysis tool according to claim 7, further comprising a substrate which supports the cover and which is provided with a plurality of electrodes.

9. The analysis tool according to claim 8, further comprising a spacer which is interposed between the substrate and the cover, and which defines the passage.
